# EUROPEAN PATENT SPECIFICATION

(11) **EP 0 922 765 B1**
(45) Date of publication and mention of the grant of the patent: **23.11.2005**
(21) Application number: 98122533.7
(22) Date of filing: 30.11.1998
(51) Int. Cl.: C12N 15/31, C12N 9/12, C12Q 1/68

(54) **Modified DNA-polymerase from carboxydothermus hydrogenoformans and its use for coupled reverse transcription and polymerase chain reaction**
Modifizierte DNA Polymerase aus Corboxydothermus hydrogenoformans und deren Verwendung für gekoppelte reverse Transkription und Polymerase-Kettenreaktion
Polymérase d'ADN modifiée de Carboxydothermus hydrogenoformans et son utilisation pour transcription inverse suivi par la réaction en chaîne de la polymérase

(30) Priority: 02.12.1997 EP 97121151
(43) Date of publication of application: 16.06.1999
(73) Proprietor: Roche Diagnostics GmbH, 68305 Mannheim (DE)
(72) Inventor: Ankenbauer, Waltraud, Dr., 82377 Penzberg (DE); Markau, Ursula, 82398 Polling (DE); Ebenbichler, Christine, 82387 Antdorf (DE); Achhammer, Gunthar, Dr., 82377 Penzberg (DE)

(56) References cited:
- EP-A- 0 834 569
- WO-A-91/09944
- WO-A-92/03556
- WO-A-96/10640
- SVETLICHNY V A ET AL: "Carboxydothermus hydrogenoformans gen. nov., sp. nov., a CO-utilizing thermophilic anaerobic bacterium from hydrothermal environments of Lunashir Island" SYSTEM. APPL. MICROBIOL., vol. 14, no. 3, 1991, pages 254-260, XP002074994

### SEQUENCE LISTING

(1) GENERAL INFORMATION:
   (i) APPLICANT:
      (A) NAME: Boehringer Mannheim GmbH
      (B) STREET: Sandhoferstr. 116
      (C) CITY: Mannheim
      (E) COUNTRY: DE
      (F) POSTAL CODE (ZIP): 68305
      (G) TELEPHONE: 06217595482
      (H) TELEFAX: 06217594457
   (ii) TITLE OF INVENTION: Modified DNA-Polymerase from carboxydothermus hydrogenformans and its use for Coupled Reverse Transcription and Polymerase Chain Reaction
   (iii) NUMBER OF SEQUENCES: 12
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 1:
   CCNAAYYTNC ARAAYATH                                                    18
(2) INFORMATION FOR SEQ ID NO: 2:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 2:
   YTCRTCRTGN ACYTG                                                       15
(2) INFORMATION FOR SEQ ID NO: 3:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 3:
   GGGCGAAGAC GCTATATTCC TGAGC 25
(2) INFORMATION FOR SEQ ID NO: 4:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 4:
   GAAGCCTTAA TTCAATCTGG GAATAATC 28
(2) INFORMATION FOR SEQ ID NO: 5:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 5:
   CGAATTCAAT CCATGGGAAA AGTAGTCCTG GTGGAT 36
(2) INFORMATION FOR SEQ ID NO: 6:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 6:
   CGAATTCAAG GATCCTTACT TCGCTTCATA CCAGTT 36
(2) INFORMATION FOR SEQ ID NO: 7:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 7:
   CGGTAAACCC ATGGTTAATT TCTCCTCTTT AATGAATTC 39
(2) INFORMATION FOR SEQ ID NO: 8:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 8:
   CGGGAATCCA TGGAAAAGCT TGCCGAACAC GAAAATTTA 39
(2) INFORMATION FOR SEQ ID NO: 9:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 9:
   AATTCGGATG GCTACGTACA TGGCTG 26
(2) INFORMATION FOR SEQ ID NO: 10:
   (i) SEQUENCE CHARACTERISTICS:
      (A) LENGTH: 1824 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION:1..1824
   (xi) SEQUENCE DESCRIPTION: SEQ ID NO: 10:
   ATG GAA AAG CTT GCC GAA CAC GAA AAT TTA GCA AAA ATA TCG AAA CAA   48
   Met Glu Lys Leu Ala Glu His Glu Asn Leu Ala Lys Ile Ser Lys Gln
    1               5                  10                  15
   TTA GCT ACA ATC CTG CGG GAA ATA CCG TTA GAA ATC TCC CTG GAA GAT   96
   Leu Ala Thr Ile Leu Arg Glu Ile Pro Leu Glu Ile Ser Leu Glu Asp
               20                  25                  30
   TTA AAA GTT AAA GAA CCT AAT TAT GAA GAA GTT GCT AAA TTA TTT CTT  144
   Leu Lys Val Lys Glu Pro Asn Tyr Glu Glu Val Ala Lys Leu Phe Leu
           35                  40                  45
   CAC CTT GAG TTT AAA AGC TTT TTA AAA GAA ATA GAA CCA AAA ATA AAG  192
   His Leu Glu Phe Lys Ser Phe Leu Lys Glu Ile Glu Pro Lys Ile Lys
       50                  55                  60
   AAA GAA TAC CAG GAA GGT AAA GAT TTG GTG CAA GTT GAA ACT GTA GAA  240
   Lys Glu Tyr Gln Glu Gly Lys Asp Leu Val Gln Val Glu Thr Val Glu
    65                  70                  75                  80
   ACG GAA GGA CAG ATT GCA GTA GTT TTT AGT GAT GGA TTT TAT GTT GAT  288
   Thr Glu Gly Gln Ile Ala Val Val Phe Ser Asp Gly Phe Tyr Val Asp
                   85                  90                  95
   GAC GGG GAA AAA ACA AAG TTT TAC TCT TTA GAC CGG CTG AAT GAA ATA  336
   Asp Gly Glu Lys Thr Lys Phe Tyr Ser Leu Asp Arg Leu Asn Glu Ile
               100                 105                 110
   GAG GAA ATA TTT AGG AAT AAA AAA ATT ATT ACC GAC GAT GCC AAA GGA  384
   Glu Glu Ile Phe Arg Asn Lys Lys Ile Ile Thr Asp Asp Ala Lys Gly
           115                 120                 125
   ATT TAT CAT GTC TGT TTA GAA AAA GGT CTG ACT TTT CCC GAA GTT TGT  432
   Ile Tyr His Val Cys Leu Glu Lys Gly Leu Thr Phe Pro Glu Val Cys
       130                 135                 140
   TTT GAT GCG CGG ATT GCA GCT TAT GTT TTA AAC CCG GCC GAC CAA AAT  480
   Phe Asp Ala Arg Ile Ala Ala Tyr Val Leu Asn Pro Ala Asp Gln Asn
   145                 150                 155                 160
   CCC GGC CTC AAG GGG CTT TAT CTA AAG TAT GAC TTA CCG GTG TAT GAA   528
   Pro Gly Leu Lys Gly Leu Tyr Leu Lys Tyr Asp Leu Pro Val Tyr Glu
                   165                 170                 175
   GAT GTA TCT TTA AAC ATT AGA GGG TTG TTT TAT TTA AAA AAA GAA ATG   576
   Asp Val Ser Leu Asn Ile Arg Gly Leu Phe Tyr Leu Lys Lys Glu Met
               180                 185                 190
   ATG AGA AAA ATC TTT GAG CAG GAG CAA GAA AGG TTA TTT TAT GAA ATA   624
   Met Arg Lys Ile Phe Glu Gln Glu Gln Glu Arg Leu Phe Tyr Glu Ile
           195                 200                 205
   GAA CTT CCT TTA ACT CCA GTT CTT GCT CAA ATG GAG CAT ACC GGC ATT   672
   Glu Leu Pro Leu Thr Pro Val Leu Ala Gln Met Glu His Thr Gly Ile
       210                 215                 220
   CAG GTT GAC CGG GAA GCT TTA AAA GAG ATG TCG TTA GAG CTG GGA GAG   720
   Gln Val Asp Arg Glu Ala Leu Lys Glu Met Ser Leu Glu Leu Gly Glu
   225                 230                 235                 240
   CAA ATT GAA GAG TTA ATC CGG GAA ATT TAT GTG CTG GCG GGG GAA GAG   768
   Gln Ile Glu Glu Leu Ile Arg Glu Ile Tyr Val Leu Ala Gly Glu Glu
                   245                 250                 255
   TTT AAC TTA AAC TCG CCC AGG CAG CTG GGA GTT ATT CTT TTT GAA AAA   816
   Phe Asn Leu Asn Ser Pro Arg Gln Leu Gly Val Ile Leu Phe Glu Lys
               260                 265                 270
   CTT GGG CTG CCG GTA ATT AAA AAG ACC AAA ACG GGC TAC TCT ACC GAT   864
   Leu Gly Leu Pro Val Ile Lys Lys Thr Lys Thr Gly Tyr Ser Thr Asp
           275                 280                 285
   GCG GAG GTT TTG GAA GAG CTC TTG CCT TTC CAC GAA ATT ATC GGC AAA   912
   Ala Glu Val Leu Glu Glu Leu Leu Pro Phe His Glu Ile Ile Gly Lys
       290                 295                 300
   ATA TTG AAT TAC CGG CAG CTT ATG AAG TTA AAA TCC ACT TAT ACT GAC   960
   Ile Leu Asn Tyr Arg Gln Leu Met Lys Leu Lys Ser Thr Tyr Thr Asp
   305                 310                 315                 320
   GGC TTA ATG CCT TTA ATA AAT GAG CGT ACC GGT AAA CTT CAC ACT ACT 1008
   Gly Leu Met Pro Leu Ile Asn Glu Arg Thr Gly Lys Leu His Thr Thr
                   325                 330                 335
   TTT AAC CAG ACC GGT ACT TTA ACC GGA CGC CTG GCG TCT TCG GAG CCC 1056
   Phe Asn Gln Thr Gly Thr Leu Thr Gly Arg Leu Ala Ser Ser Glu Pro
               340                 345                 350
   AAT CTC CAA AAT ATT CCC ATC CGG TTG GAA CTC GGT CGG AAA TTA CGC 1104
   Asn Leu Gln Asn Ile Pro Ile Arg Leu Glu Leu Gly Arg Lys Leu Arg
           355                 360                 365
   AAG ATG TTT ATA CCT TCA CCG GGG TAT GAT TAT ATT GTT TCG GCG GAT 1152
   Lys Met Phe Ile Pro Ser Pro Gly Tyr Asp Tyr Ile Val Ser Ala Asp
       370                 375                 380
   TAT TCC CAG ATT GAA TTA AGG CTT CTT GCC CAT TTT TCC GAA GAG CCC 1200
   Tyr Ser Gln Ile Glu Leu Arg Leu Leu Ala His Phe Ser Glu Glu Pro
   385                 390                 395                 400
   AAG CTT ATT GAA GCT TAC CAA AAA GGG GAG GAT ATT CAC CGG AAA ACG 1248
   Lys Leu Ile Glu Ala Tyr Gln Lys Gly Glu Asp Ile His Arg Lys Thr
                   405                 410                 415
   GCC TCC GAG GTG TTC GGT GTA TCT TTG GAA GAA GTT ACT CCC GAG ATG 1296
   Ala Ser Glu Val Phe Gly Val Ser Leu Glu Glu Val Thr Pro Glu Met
               420                 425                 430
   CGC GCT CAT GCC AAG TCG GTG AAC TTC GGC ATT GTT TAT GGC ATT AGT 1344
   Arg Ala His Ala Lys Ser Val Asn Phe Gly Ile Val Tyr Gly Ile Ser
           435                 440                 445
   GAT TTT GGT TTA GGC AGA GAC TTA AAG ATT CCC CGG GAG GTT GCC GGT 1392
   Asp Phe Gly Leu Gly Arg Asp Leu Lys Ile Pro Arg Glu Val Ala Gly
   450 455 460
   AAG TAC ATT AAA AAT TAT TTT GCC AAC TAT CCC AAA GTG CGG GAG TAT 1440
   Lys Tyr Ile Lys Asn Tyr Phe Ala Asn Tyr Pro Lys Val Arg Glu Tyr
   465 470 475 480
   CTC GAT GAA CTT GTC CGT ACG GCA AGA GAA AAG GGA TAT GTG ACC ACT 1488
   Leu Asp Glu Leu Val Arg Thr Ala Arg Glu Lys Gly Tyr Val Thr Thr
   485 490 495
   TTA TTT GGG CGA AGA CGC TAT ATT CCT GAG CTA TCT TCA AAA AAC CGC 1536
   Leu Phe Gly Arg Arg Arg Tyr Ile Pro Glu Leu Ser Ser Lys Asn Arg
   500 505 510
   ACG GTT CAG GGT TTT GGC GAA AGG ACG GCC ATG AAT ACT CCC CTT CAG 1584
   Thr Val Gln Gly Phe Gly Glu Arg Thr Ala Met Asn Thr Pro Leu Gln
   515 520 525
   GGC TCG GCT GCC GAT ATT ATT AAG CTT GCA ATG ATT AAT GTA GAA AAA 1632
   Gly Ser Ala Ala Asp Ile Ile Lys Leu Ala Met Ile Asn Val Glu Lys
   530 535 540
   GAA CTT AAA GCC CGT AAG CTT AAG TCC CGG CTC CTT CTT TCG GTG CAC 1680
   Glu Leu Lys Ala Arg Lys Leu Lys Ser Arg Leu Leu Leu Ser Val His
   545 550 555 560
   GAT GAG TTA GTT TTA GAA GTG CCG GCG GAA GAG CTG GAA GAG GTA AAA 1728
   Asp Glu Leu Val Leu Glu Val Pro Ala Glu Glu Leu Glu Glu Val Lys
   565 570 575
   GCG CTG GTA AAA GGG GTT ATG GAG TCG GTG GTT GAA CTG AAA GTG CCT 1776
   Ala Leu Val Lys Gly Val Met Glu Ser Val Val Glu Leu Lys Val Pro
   580 585 590
   TTA ATC GCT GAA GTT GGT GCA GGC AAA AAC TGG TAT GAA GCG AAG TAA 1824
   Leu Ile Ala Glu Val Gly Ala Gly Lys Asn Trp Tyr Glu Ala Lys  *
           595                 600                 605
(2) INFORMATION FOR SEQ ID NO: 11:
   (i) SEQUENCE CHARACTERISTICS:
      (A) LENGTH: 607 amino acids
      (B) TYPE: amino acid
      (D) TOPOLOGY: linear
   (ii) MOLECULE TYPE: protein
   (xi) SEQUENCE DESCRIPTION: SEQ ID NO: 11:
   Met Glu Lys Leu Ala Glu His Glu Asn Leu Ala Lys Ile Ser Lys Gln
     1               5                  10                  15
   Leu Ala Thr Ile Leu Arg Glu Ile Pro Leu Glu Ile Ser Leu Glu Asp
                20                  25                  30
   Leu Lys Val Lys Glu Pro Asn Tyr Glu Glu Val Ala Lys Leu Phe Leu
            35                  40                  45
   His Leu Glu Phe Lys Ser Phe Leu Lys Glu Ile Glu Pro Lys Ile Lys
        50                  55                  60
   Lys Glu Tyr Gln Glu Gly Lys Asp Leu Val Gln Val Glu Thr Val Glu
    65                  70                  75                      80
   Thr Glu Gly Gln Ile Ala Val Val Phe Ser Asp Gly Phe Tyr Val Asp
                    85                  90                  95
   Asp Gly Glu Lys Thr Lys Phe Tyr Ser Leu Asp Arg Leu Asn Glu Ile
               100                 105                 110
   Glu Glu Ile Phe Arg Asn Lys Lys Ile Ile Thr Asp Asp Ala Lys Gly
           115                 120                 125
   Ile Tyr His Val Cys Leu Glu Lys Gly Leu Thr Phe Pro Glu Val Cys
   130 135 140
   Phe Asp Ala Arg Ile Ala Ala Tyr Val Leu Asn Pro Ala Asp Gln Asn
   145 150 155 160
   Pro Gly Leu Lys Gly Leu Tyr Leu Lys Tyr Asp Leu Pro Val Tyr Glu
   165 170 175
   Asp Val Ser Leu Asn Ile Arg Gly Leu Phe Tyr Leu Lys Lys Glu Met
   180 185 190
   Met Arg Lys Ile Phe Glu Gln Glu Gln Glu Arg Leu Phe Tyr Glu Ile
   195 200 205
   Glu Leu Pro Leu Thr Pro Val Leu Ala Gln Met Glu His Thr Gly Ile
   210 215 220
   Gln Val Asp Arg Glu Ala Leu Lys Glu Met Ser Leu Glu Leu Gly Glu
   225 230 235 240
   Gln Ile Glu Glu Leu Ile Arg Glu Ile Tyr Val Leu Ala Gly Glu Glu
   245 250 255
   Phe Asn Leu Asn Ser Pro Arg Gln Leu Gly Val Ile Leu Phe Glu Lys
   260 265 270
   Leu Gly Leu Pro Val Ile Lys Lys Thr Lys Thr Gly Tyr Ser Thr Asp
   275 280 285
   Ala Glu Val Leu Glu Glu Leu Leu Pro Phe His Glu Ile Ile Gly Lys
   290 295 300
   Ile Leu Asn Tyr Arg Gln Leu Met Lys Leu Lys Ser Thr Tyr Thr Asp
   305 310 315 320
   Gly Leu Met Pro Leu Ile Asn Glu Arg Thr Gly Lys Leu His Thr Thr
   325 330 335
   Phe Asn Gln Thr Gly Thr Leu Thr Gly Arg Leu Ala Ser Ser Glu Pro
   340 345 350
   Asn Leu Gln Asn Ile Pro Ile Arg Leu Glu Leu Gly Arg Lys Leu Arg
   355 360 365
   Lys Met Phe Ile Pro Ser Pro Gly Tyr Asp Tyr Ile Val Ser Ala Asp
   370 375 380
   Tyr Ser Gln Ile Glu Leu Arg Leu Leu Ala His Phe Ser Glu Glu Pro
   385 390 395 400
   Lys Leu Ile Glu Ala Tyr Gln Lys Gly Glu Asp Ile His Arg Lys Thr
   405 410 415
   Ala Ser Glu Val Phe Gly Val Ser Leu Glu Glu Val Thr Pro Glu Met
   420 425 430
   Arg Ala His Ala Lys Ser Val Asn Phe Gly Ile Val Tyr Gly Ile Ser
   435 440 445
   Asp Phe Gly Leu Gly Arg Asp Leu Lys Ile Pro Arg Glu Val Ala Gly
   450 455 460
   Lys Tyr Ile Lys Asn Tyr Phe Ala Asn Tyr Pro Lys Val Arg Glu Tyr
   465 470 475 480
   Leu Asp Glu Leu Val Arg Thr Ala Arg Glu Lys Gly Tyr Val Thr Thr
   485 490 495
   Leu Phe Gly Arg Arg Arg Tyr Ile Pro Glu Leu Ser Ser Lys Asn Arg
   500 505 510
   Thr Val Gln Gly Phe Gly Glu Arg Thr Ala Met Asn Thr Pro Leu Gln
           515                 520                 525
   Gly Ser Ala Ala Asp Ile Ile Lys Leu Ala Met Ile Asn Val Glu Lys
       530                 535                 540
   Glu Leu Lys Ala Arg Lys Leu Lys Ser Arg Leu Leu Leu Ser Val His
   545                 550                 555                 560
   Asp Glu Leu Val Leu Glu Val Pro Ala Glu Glu Leu Glu Glu Val Lys
                   565                 570                 575
   Ala Leu Val Lys Gly Val Met Glu Ser Val Val Glu Leu Lys Val Pro
               580                 585                 590
   Leu Ile Ala Glu Val Gly Ala Gly Lys Asn Trp Tyr Glu Ala Lys
           595                 600                 605
(2) INFORMATION FOR SEQ ID NO: 12:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (xi) SEQUENCE DESCRIPTION: SEQ ID NO: 12:
   AGCTTGCTGT ATTCCCCTCC ATCGTG                                      26

## Description

The present invention relates to a modified DNA-polymerase having reverse transcriptase activity and reduced 5'-3' exonuclease activity derived from a native polymerase which is obtainable from *Carboxydothermus hydrogenoformans*.
Furthermore the invention relates to the field of molecular biology and provides methods for amplifying a DNA segment from an RNA template using an enzyme with reverse transcriptase activity (RT-PCR). In another aspect, the invention provides a kit for Coupled High Temperature Reverse Transcription and Polymerase Chain Reaction.

Heat stable DNA polymerases (EC 2.7.7.7. DNA nucleotidyltransferase, DNA-directed) have been isolated from numerous thermophilic organisms (for example: Kaledin et al. (1980), *Biokhimiya* **45**, 644-651; Kaledin et al. (1981) *Biokhimiya* **46**, 1576-1584; Kaledin et al. (1982) *Biokhimiya* **47**, 1785-1791; Ruttimann et al. (1985) *Eur. J. Biochem.* **149**, 41-46; Neuner et al. (1990) *Arch. Microbiol.* **153**, 205-207).
For some organisms, the polymerase gene has been cloned and expressed (Lawyer et al. (1989) *J. Biol. Chem.* **264**, 6427-6437; Engelke et al. (1990) *Anal. Biochem.* **191**, 396-400; Lundberg et al. (1991) *Gene* **108**, 1-6; Perler et al. (1992) *Proc. Natl. Acad. Sci.* USA **89**, 5577-5581).

Thermophilic DNA polymerases are increasingly becoming important tools for use in molecular biology and there is growing interest in finding new polymerases which have more suitable properties and activities for use in diagnostic detection of RNA and DNA, gene cloning and DNA sequencing. At present, the thermophilic DNA polymerases mostly used for these purposes are from Thermus species like Taq polymerase from *T. aquaticus* (Brock et al. (1969) *J. Bacteriol.* **98,** 289-297)

The term "reverse transcriptase" describes a class of polymerases characterized as RNA-dependent DNA-polymerases. All known reverse transcriptases require a primer to synthesize a DNA-transcript from an RNA template. Historically, reverse transcriptase has been used primarily to transcribe mRNA into cDNA which can then be cloned into a vector for further manipulation.

Reverse transcription is commonly performed with viral reverse transcriptases like the enzymes isolated from *Avian myeloblastosis* virus or *Moloney murine leukemia* virus. Both enzymes mentioned are active in the presence of magnesium ions but have the disadvantages to possess RNase H-activity, which destroys the template RNA during the reverse transcription reaction and have a temperature optimum at 42°C or 37°C, respectively. Avian myoblastosis virus (AMV) reverse transcriptase was the first widely used RNA-dependent DNA-polymerase (Verma (1977) *Biochem. Biophys. Acta* **473**, 1). The enzyme has 5'-3' RNA-directed DNA polymerase activity, 5'-3' DNA directed DNA polymerase activity, and RNaseH activity. RNaseH is a processive 5'-3' ribonuclease specific for the RNA strand of RNA-DNA hybrids (Perbal (1984), A Practical Guide to Molecular Cloning, Wiley & Sons New York). Errors in transcription cannot be corrected because known viral reverse transcriptases lack the 3'-5' exonuclease activity necessary for proofreading (Saunders and Saunders (1987) Microbial Genetics Applied to Biotechnology, Croom Helm, London). A detailed study of the activity of AMV reverse transcriptase and its associated RNaseH activity has been presented by Berger et al., (1983) *Biochemistry* **22**, 2365-2372.

DNA polymerases isolated from mesophilic microorganisms such as *E. coli* have been extensively characterized (see, for example, Bessmann et al. (1957) *J. Biol. Chem.* **233**, 171-177 and Buttin and Kornberg (1966) *J. Biol. Chem.* **241,** 5419-5427). *E. coli* DNA polymerase I (Pol I) is useful for a number of applications including: nick-translation reactions, DNA sequencing, in vitro mutagenesis, second strand cDNA synthesis, polymerase chain reactions (PCR), and blunt end formation for linker ligation (Maniatis et al., (1982) Molecular Cloning: A Laboratory Manual Cold Spring Harbor, New York).

Several laboratories have shown that some polymerases are capable of *in vitro* reverse transcription of RNA (Karkas (1973) *Proc. Nat. Acad Sci.* USA **70**, 3834-3838; Gulati et al. (1974) *Proc. Nat. Acad. Sci.* USA **71**, 1035-1039; and Wittig and Wittig, (1978) *Nuc. Acids Res.* **5**, 1165-1178). Gulati et al. found that *E. coli* Pol I could be used to transcribe Qβ viral RNA using oligo(dT)₁₀ as a primer. Wittig and Wittig have shown that *E.coli* Pol I can be used to reverse transcribe tRNA that has been enzymatically elongated with oligo(dA). However, as Gulati et aL demonstrated, the amount of enzyme required and the small size of cDNA product suggest that the reverse transcriptase activity of *E. coli* Pol I has little practical value.

Alternative methods are described using the reverse transcriptase activity of DNA polymerases of thermophilic organisms which are active at higher temperatures. Reverse transcription at higher temperatures is of advantage to overcome secondary structures of the RNA template which could result in premature termination of products. Thermostable DNA polymerases with reverse transcriptase activities are commonly isolated from Thermus species. These DNA polymerases however, show reverse transcriptase activity only in the presence of manganese ions. These reaction conditions are suboptimal, because in the presence of manganese ions the polymerase copies the template RNA with low fidelity.

Another feature of the commonly used reverse transcriptases is that they do not contain 3'-5' exonuclease activity. Therefore, misincorporated nucleotides cannot be removed and thus the cDNA copies from the template RNA may contain a significant degree of mutations.

One of the known DNA polymerases having high reverse transcriptase activity is obtainable from *Thermus thermophilus* (Tth polymerase) (WO 91/09944). Tth polymerase, as well as Taq polymerase, lacks 3' to 5' exonucleolytic proofreading activity. This 3' to 5' exonuclease activity is generally considered to be desirable because it allows removal of misincorporated or unmatched bases in the newly synthesized nucleic acid sequences. Another thermophilic pol I-type DNA polymerase isolated from *Thermotoga maritima* (Tma pol) has 3' to 5' exonuclease activity. U.S. patent 5,624,833 provides means for isolating and producing Tma polymerase. Still another DNA polymerase from Thermotoga neapolitana (Tne pol) has substantially reduced 3' to 5' exonuclease or none of such activity at all (WO 96/10640). However, all those DNA polymerases, Tth as well as Tma and Tne polymerase, show useful reverse transcriptase activity only in the presence of manganese ions.

The DNA polymerase of *Carboxydothermus hydrogenoformans* shows reverse transcription activity in the presence of magnesium ions and in the substantial absence of manganese ions and can be used to reverse transcribe RNA, to detect and amplify (in combination with a thermostable DNA polymerase like Taq) specific sequences of RNA. Using DNA polymerase of *Carboxydothermus hydrogenoformans* polymerase a high specificity of transcription is observed with short incubation times. A high specificity is observed using e.g. 5 min of incubation time and 33 units of DNA polymerase protein. With longer incubation times also with lower amounts of *Carboxydothermus hydrogenoformans* polymerase specific products can be obtained. However an unspecific smear of products is occurring. These unspecific products might be caused by the 5'-3' exonuclease activity of the polymerase which enables the enzyme to cleave the template at secondary structures ("RNaseH"-activity) and to create additional primers which can be elongated by the DNA polymerase activity. The thermostable DNA polymerase from *Carboxydothermus hydrogenoformans* has been identified and cloned and is described in the copending European application with the Application No. 96115873.0, filed October 03, 1996, and incorporated herein by reference.

In summary, reverse transcriptases as MoMULV-RT or AMV-RT perform reverse transcription in the presence of magnesium-ions. However, these enzymes act at temperatures between 37°C and 55°C. Reverse transcription at higher temperatures would be desirable because secondary structures can be overcome in the template in order to avoid premature termination of the reaction and to assure the production of cDNA without deletions.
Other enzymes e.g. DNA polymerase obtainable from *Thermus spec*. act as reverse transcriptase at temperatures up to 70°C in the presence of manganese ions. These reaction conditions are suboptimal, because in the presence of manganese ions the polymerase copies the template RNA with low fidelity and the RNA strand will be degraded. Degradation of the RNA strand occurs faster in the presence of manganese ions as in the presence of magnesium ions. Therefore, if manganese ions are present complexation of the manganese ions (e.g. with EDTA) is required after cDNA synthesis in order to obtain a higher fidelity during cDNA amplification in the subsequent PCR reaction.

Therefore, it is desirable to develop a reverse transcriptase
- which acts at higher temperatures to overcome secondary structures in the template to avoid premature termination of the reaction and to assure the production of cDNA without deletions
- which is active in the presence of magnesium ions in order to prepare cDNA from RNA templates with higher fidelity and
- which has 3*'*-5*'*-exonuclease in order to remove misincorporated nucleotides before continuation of DNA synthesis and to produce products with low mutation frequency
- which has a high specificity and produces exclusively or predominantly RT-PCR products derived from specific primer binding.

The present invention addresses these needs and provides a DNA polymerase mutant active at higher temperatures which has reverse transcriptase activity in the presence of magnesium ions and which has 3*'*-5*'* exonuclease activity and reduced or no 5'-3' exonuclease activity.

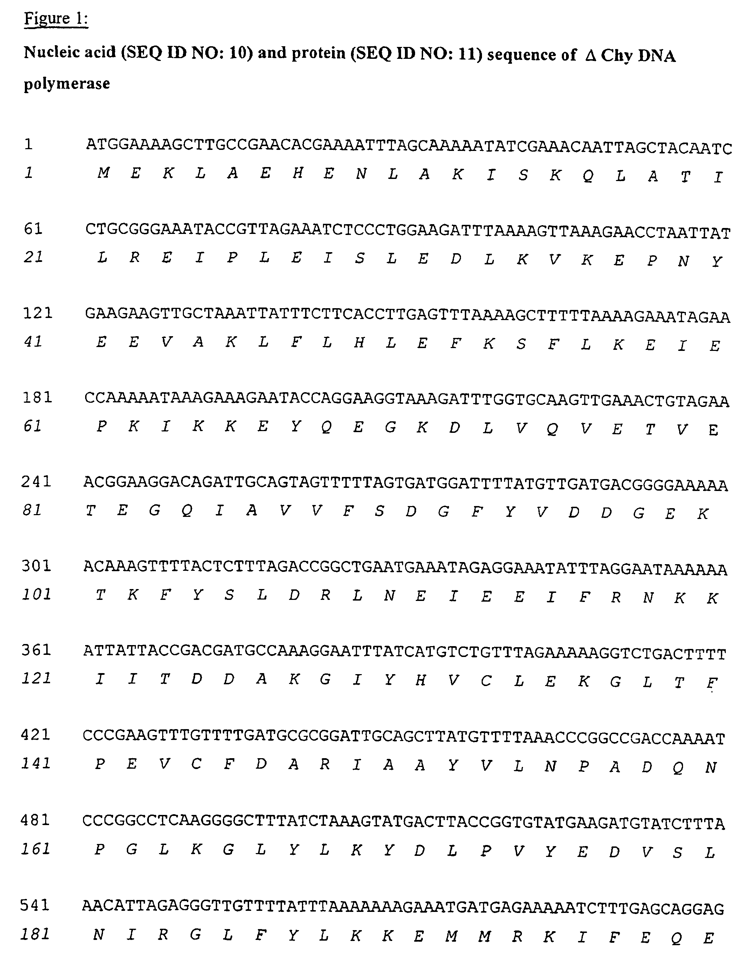

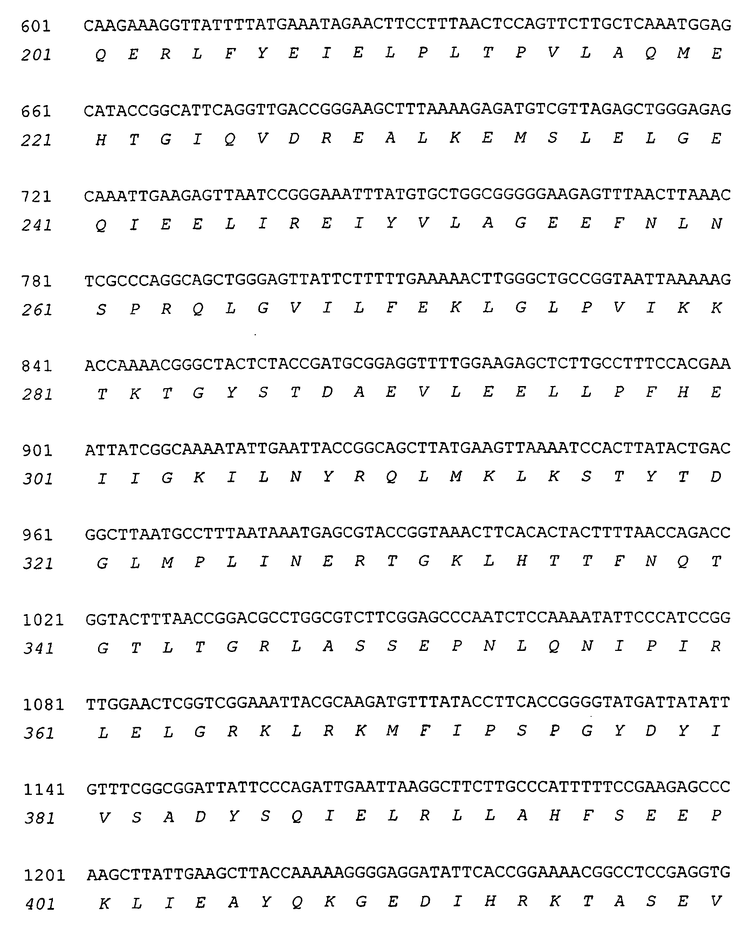

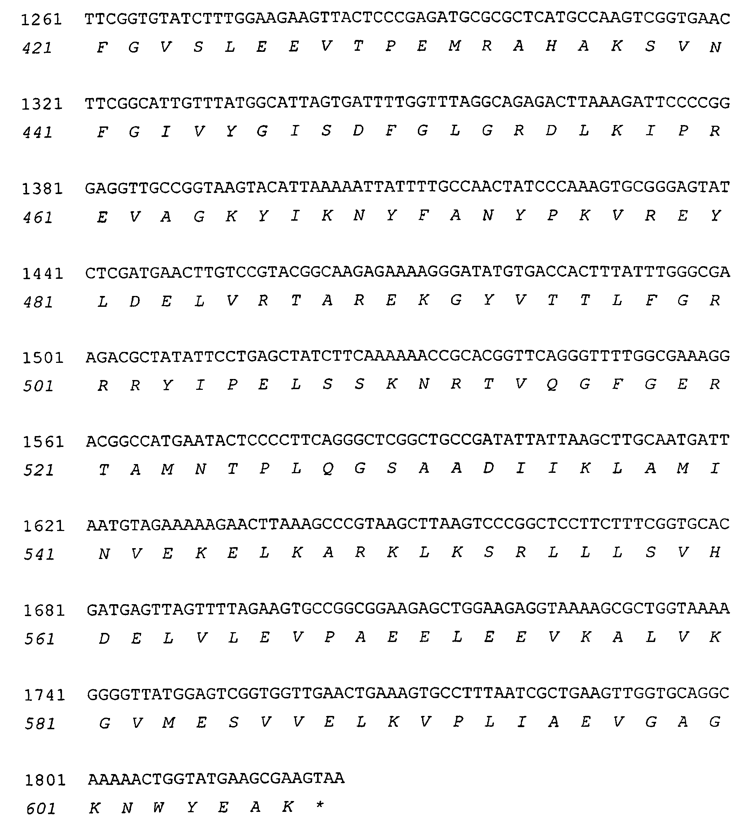

It is an object of this invention to provide a polymerase enzyme (EC 2.7.7.7.), characterized in that it has reverse transcriptase activity in the presence of magnesium ions as well as in the presence of manganese ions. In a further aspect the invention comprises a DNA polymerase having 3*'*-5*'*-exonuclease activity and reduced 5'-3' exonuclease activity. The enzyme according to the invention can be obtained from a polymerase obtainable from *Carboxydothermus hydrogenoformans* (Deutsche Sammlung von Mikroorganismen und Zellkulturen GmbH, Mascheroder Weg 1b, D-38124 Braunschweig, DSM No. 8979). In a further aspect the invention is directed to a DNA polymerase with reduced 5'-3' exonuclease activity having reverse transcriptase activity in the presence of magnesiums ions and in the substantial absence of manganese ions. In a further aspect the invention comprises a DNA polymerase having a molecular mass of about 64 to 71 kDa as determined by SDS PAGE analysis. The mutant polymerase enzyme with reduced 5'-3' exonuclease activity derived from a polymerase obtainable from *Carboxidothermus hydrogenoformans* is called hereinafter Δ Chy Polymerase. In a further aspect the invention comprises a recombinant DNA sequence that encodes DNA polymerase activity of the Δ Chy Polymerase. In a related aspect, the DNA sequence is depicted as SEQ ID No. 10 (Figure 1). In a second related aspect the invention comprises a recombinant DNA sequence that encodes essentially amino acid residues 1 to 607 (SEQ ID No. 11, Figure 1). In a further aspect the invention comprises a recombinant DNA plasmid that comprises the DNA sequence of the invention inserted into plasmid vectors and which can be used to drive the expression of the Δ Chy DNA polymerase in a host cell transformed with the plasmid. In a further aspect the invention includes a recombinant strain comprising the vector pDS56 carrying the Δ Chy DNA polymerase gene and designated pΔ₂₋₂₂₅AR₄. The E.coli strain XL1 carrying the plasmid pΔ₂₋₂₂₅AR₄ was deposited on the Deutsche Sammlung von Mikroorganismen und Zellkulturen GmbH, Mascherorder Weg 1b, D-38124 Braunschweig DSM No. 11854 (BMTU 7307) is designated *E.coli* GA1.

In referring to a peptide chain as being comprised of a series of amino acids "substantially or effectively" in accordance with a list offering no alternatives within itself, we include within that reference any versions of the peptide chain bearing substitutions made to one or more amino acids in such a way that the overall structure and the overall function of the protein composed of that peptide chain is substantially the same as - or undetectably different to - that of the unsubstituted version. For example it is generally possible to exchange alanine and valine without greatly changing the properties of the protein, especially if the changed site or sites are at positions not critical to the morphology of the folded protein.

3'-5' exonuclease activity is commonly referred as "proofreading" or "editing" activity of DNA polymerases. It is located in the small domain of the large fragment of Type A polymerases. This activity removes mispaired nucleotides from the 3' end of the primer terminus of DNA in the absence of nucleoside triphosphates (Kornberg A. and Baker T. A.(1992) DNA Replication W. H. Freemann & Company, New York). This nuclease action is suppressed by deoxynucleoside triphosphates if they match to the template and can be incorporated into the polymer.

The 3'-5' exonuclease activity of the claimed DNA polymerase can be measured as degradation or shortening of a 5'-digoxygenin-labeled oligonucleotide annealed to template DNA in the absence or presence of deoxyribonucleoside triphosphates or on DNA fragments in the absence or presence of deoxyribonucleoside triphosphates.

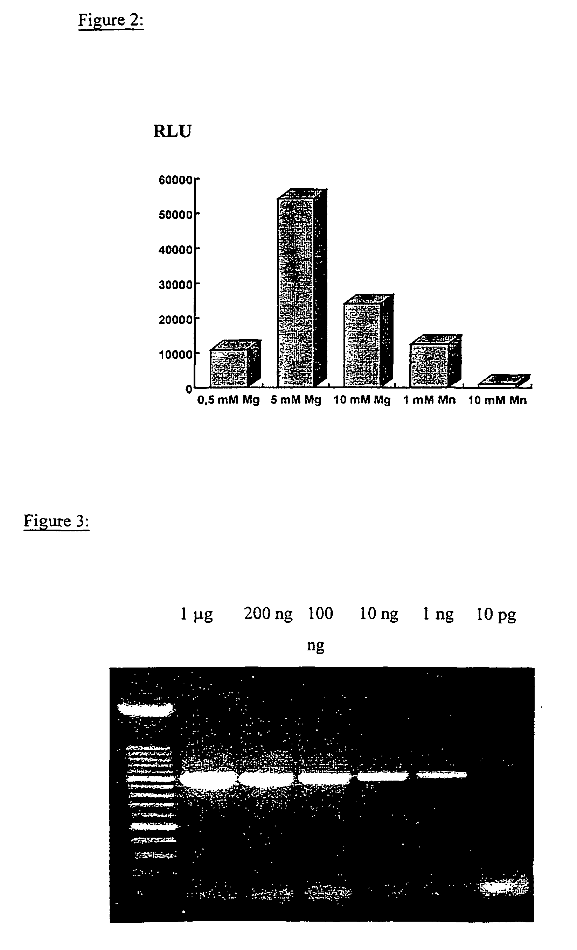

*Carboxydothermus hydrogenoformans* DNA polymerase is the first DNA polymerase isolated from thermophilic eubacteria with a higher activity in the presence of magnesium ions than in the presence of manganese ions as shown in figure 2. The reverse transcriptase activity in dependence of magnesium is of advantage since the DNA polymerases synthesize DNA with higher fidelity in the presence of magnesium than in the presence of manganese (Beckmann R. A. et al. (1985) *Biochemistry* **24**, 5810-5817; Ricchetti M. and Buc H. (1993) *EMBO J.* **12**, 387-396). Low fidelity DNA synthesis is likely to lead to mutated copies of the original template. In addition, Mn²⁺ ions have been implicated in an increased rate of RNA degradation, particularly at higher temperatures and this can cause the synthesis of shortened products in the reverse transcription reaction.

The DNA sequence (SEQ ID No.: 10) Δ Chy polymerase and the derived amino acid sequence (SEQ ID No.: 11) of the enzyme are shown in figure 1. The molecular weight deduced from the sequence is 70,3 kDa, in SDS polyacrylamide gel electrophoresis however Δ Chy polymerase has an electrophoretic mobility of approx. 65 kDa.

The Δ Chy DNA Polymerase has reduced 5'-3' - exonuclease activity and has a temperature optimum at 72°C and exhibits reverse transcriptase activity at temperatures between 50 °C and 75 °C.

When using Δ Chy DNA Polymerase obtainable from *Carboxydothermus hydrogenoformans* having reduced 5'-3' - exonuclease activity in RT-PCR as reverse transcriptase with subsequent PCR reaction using Taq-polymerase as PCR enzyme a remarkable high sensitivity is achieved (Figure 3). The sensitivity of Δ Chy DNA Polymerase in RT-PCR is higher than the sensitivity of e.g. DNA polymerase from *Thermus thermophilus* (Tth polymerase) (Example 3, Figure 4). Δ Chy DNA Polymerase also exhibits high sensitivity by amplifying a 1.83 kB fragment from total RNA from human muscle (Figure 5). The error rate of Δ Chy DNA Polymerase is 1,58 x 10⁻⁴ mutations per nucleotide per cycle and is therewith lower than the error rate of Tth Polymerase which is 2.37 x 10⁻⁴ mutations per nucleotide per cycle. This results in higher fidelity of Δ Chy DNA polymerase in comparison to Tth Polymerase.

*Carboxydothermus hydrogenoformans* was isolated from a hot spring in Kamchatka by V. Svetlichny. A sample of *C. hydrogenoformans* was deposited on the Deutsche Sammlung von Mikroorganismen und Zellkulturen GmbH (DSM) under the terms of the Budapest Treaty and received Accession Number DSM 8979. The thermostable polymerase isolated from Carboxydothermus hydrogenoformans has a molecular weight of 100 to 105 KDa. The thermostable enzyme possesses 5*'*-3*'* polymerase activity, a 3*'*-5*'*- exonuclease activity and a reverse transcriptase-activity which is Mg⁺⁺-dependent. The thermostable enzyme may be native or recombinant and may be used for first- and second-strand cDNA synthesis, in cDNA cloning, DNA sequencing, DNA labeling and DNA amplification.

For recovering the native protein *C.hydrogenoformans* may be grown using any suitable technique, such as the technique described by Svetlichny et al. (1991) *System. Appl. Microbiol.* **14**, 205-208. After cell growth one preferred method for isolation and purification of the enzyme is accomplished using the multi-step process as follows:

The cells are thawed, suspended in buffer A (40 mM Tris-HCl, pH 7.5, 0.1 mM EDTA, 7 mM 2-mercaptoethanol, 0.4 M NaCl, 10 mM Pefabloc) and lysed by twofold passage through a Gaulin homogenizer. The raw extract is cleared by centrifugation, the supernatant dialyzed against buffer B (40 mM Tris-HCl, pH 7.5, 0.1 mM EDTA, 7 mM 2-mercaptoethanol, 10 % Glycerol) and brought onto a column filled with Heparin-Sepharose (Pharmacia). In each case the columns are equilibrated with the starting solvent and after the application of the sample washed with the threefold of its volume with this solvent. Elution of the first column is performed with a linear gradient of 0 to 0.5 M NaCl in Buffer B. The fractions showing polymerase activity are pooled and ammonium sulfate is added to a final concentration of 20 %. This solution is applied to a hydrophobic column containing Butyl-TSK-Toyopearl (TosoHaas). The column is eluted with a falling gradient of 20 to 0 % ammonium sulfate. The pool containing the activity is dialysed and again transferred to a column of DEAE-Sepharose (Pharmacia) and eluted with a linear gradient of 0-0.5 M NaCl in buffer B. The fourth column contains Tris-Acryl-Blue (Biosepra) and is eluted as in the preceding case. Finally the active fractions are dialyzed against buffer C (20 mM Tris-HCl, pH 7.5, 0.1 mM EDTA, 7.0 mM 2-mercaptoethanol, 100 mM NaCl, 50 % Glycerol.

DNA polymerase activity was measured by incorporation of digoxigenin-labeled dUTP into the synthesized DNA and detection and quantification of the incorporated digoxigenin essentially according to the method described in Höltke, H.-J.; Sagner, G; Kessler, C. and Schmitz, G. (1992) *Biotechniques* **12**, 104 -113. The reaction is performed in a reaction volume of 50 µl containing 1 or 2 µl of diluted (0.05 U - 0.01 U) DNA polymerase and 50 mM Tris-HCl, pH 8.5; 12.5 mM (NH₄)₂SO₄; 10 mM KCl; 5 mM MgCl₂; 10 mM 2-mercaptoethanol; 33 µM dNTPs; 200 µg/ml BSA; 12 µg of DNAse I-activated DNA from calf thymus and 0.036 µM digoxigenin-dUTP.

The samples are incubated for 30 min. at 72°C, the reaction is stopped by addition of 2 µl 0.5 M EDTA, and the tubes placed on ice. After addition of 8 µl 5 M NaCl and 150 µl of Ethanol (precooled to -20°C) the DNA is precipitated by incubation for 15 min. on ice and pelleted by centrifugation for 10 min at 13000 x rpm and 4°C. The pellet is washed with 100 µl of 70% Ethanol (precooled to -20°C) and 0.2 M NaCl, centrifuged again and dried under vacuum.

The pellets are dissolved in 50 µl Tris-EDTA (10 mM/0.1 mM; pH 7.5). 5 µl of the sample are spotted into a well of a nylon membrane bottomed white microwave plate (Pall Filtrationstechnik GmbH, Dreieich, FRG, product no: SM045BWP). The DNA is fixed to the membrane by baking for 10 min. at 70°C. The DNA loaded wells are filled with 100 µl of 0.45 µm-filtrated 1 % blocking solution (100 mM maleic acid, 150 mM NaCl, 1 % (w/v) casein, pH 7.5). All following incubation steps are done at room temperature. After incubation for 2 min. the solution is sucked through the membrane with a suitable vacuum manifold at -0.4 bar. After repeating the washing step, the wells are filled with 100 µl of a 1:10 000-dilution of Anti-digoxigenin-AP, Fab fragments (Boehringer Mannheim, FRG, no: 1093274) diluted in the above blocking solution. After incubation for 2 min. and sucking this step is repeated once. The wells are washed twice under vacuum with 200 µl each time washing-buffer 1 (100 mM maleic-acid, 150 mM NaCl, 0.3 %(v/v) Tween™ 20, pH 7.5). After washing another two times under vacuum with 200 µl each time washing-buffer 2 (10 mM Tris-HCl, 100 mM NaCl, 50 mM MgCl₂, pH 9.5) the wells are incubated for 5 min. with 50 µl of CSPD™ (Boehringer Mannheim, no: 1655884), diluted 1 : 100 in washing-buffer 2, which serves as a chemiluminescent substrate for the alkaline phosphatase. The solution is sucked through the membrane and after 10 min. incubation the RLU/s (Relative Light Unit per second) are detected in a Luminometer e.g. MicroLumat LB 96 P (EG&G Berthold, Wilbad, FRG).

With a serial dilution of *Taq* DNA polymerase a reference curve is prepared from which the linear range serves as a standard for the activity determination of the DNA polymerase to be analyzed.

The Determination of reverse transcriptase activity is performed essentially as described for determination of DNA polymerase activity except that the reaction mixture consists of the following components: 1 µg of polydA-(dT)₁₅, 33 µM of dTTP, 0.36 µM of digoxigenin-dUTP, 200 mg/ml BSA, 10 mM Tris-HCl, pH 8.5, 20 mM KCl, 5 mM MgCl₂, 10 mM DTE and various amounts of DNA polymerase The incubation temperature used is 50°C.

Isolation of recombinant DNA polymerase from *Carboxydothermus hydrogenoformans* may be performed with the same protocol or with other commonly used procedures.

The production of a recombinant form of *Carboxydothermus hydrogenoformans* DNA polymerase generally includes the following steps: chromosomal DNA from *Carboxydothermus hydrogenoformans* is isolated by treating the cells with detergent e.g. SDS and a proteinase e.g. Proteinase K. The solution is extracted with phenol and chloroform and the DNA purified by precipitation with ethanol. The DNA is dissolved in Tris/EDTA buffer and the gene encoding the DNA polymerase is specifically amplified by the PCR technique using two mixed oligonucleotides (primer 1 and 2). These oligonucleotides, described by SEQ ID No.: 1 and SEQ ID No.: 2, were designed on the basis of conserved regions of family A DNA polymerases as published by Braithwaite D. K. and Ito J. (1993) *Nucl. Acids Res.* **21**, 787 - 802. The specifically amplified fragment is ligated into an vector, preferably the pCR™II vector (Invitrogen) and the sequence is determined by cycle-sequencing. Complete isolation of the coding region and the flanking sequences of the DNA polymerase gene can be performed by restriction fragmentation of the *Carboxydothermus hydrogenoformans* DNA with another restriction enzyme as in the first round of screening and by inverse PCR (Innis et al., (1990) PCR Protocols; Academic Press, Inc., 219-227). This can be accomplished with synthesized oligonucleotide primers binding at the outer DNA sequences of the gene part but in opposite orientation. These oligonucleotides described by SEQ ID Nos. 3 and 4, were designed on the basis of the sequences which were determined by sequencing of the first PCR product described above. As template DNA from *Carboxydothermus hydrogenoformans* is used which is cleaved by restriction digestion and circularized by contacting with T4 DNA ligase. To isolate the coding region of the entire polymerase gene, another PCR is performed using primers as shown in SEQ ID Nos. 5 and 6. The complete DNA polymerase gene is amplified directly from genomic DNA with primers suitable for introducing ends compatible with the linearized expression vector.
SEQ ID No. 1: SEQ ID No. 2: SEQ ID No. 3: SEQ ID NO. 4: SEQ ID NO. 5: SEQ ID NO. 6:

The gene is operably linked to appropriate control sequences for expression in either prokaryotic or eucaryotic host/vector systems. The vector preferably encodes all functions required for transformation and maintenance in a suitable host, and may encode selectable markers and/or control sequences for polymerase expression. Active recombinant thermostable polymerase can be produced by transformed host cultures either continuously or after induction of expression. Active thermostable polymerase can be recovered either from host cells or from the culture media if the protein is secreted through the cell membrane.

The use of a plasmid as an appropriate vector has shown to be advantageously, particularly pDS56 (Stüber, D., Matile, H. and Garotta, G. (1990) Immunological Methods, Letkovcs, I. and Pernis, B., eds). The plasmid carrying the Carboxydothermus hydrogenoformans DNA polymerase gene is then designated pAR4.

According to the present invention the use of the E. coli strain BL21 (DE3) pUBS520 (Brinkmann et al., (1989) Gene 85, 109-114) has shown to be advantageously. The E.coli strain BL 21 (DEB) pUBS 520 transformed with the plasmid pAR4 is then designated AR96 (DSM No 11179).

The mutant ΔChy was obtained by deletion of an N-terminal fragment of the recombinant wild type *Carboxydothermus hydrogenoformans* DNA polymerase using inverse PCR (Innis et al., (1990) PCR Protocols; Academic Press, Inc., p 219-227). The reverse primer used is complementary to the cloning site of the expression vector pDS56 (Stüber, D., Matile, H. and Garotta, G. (1990) Immunological Methods, Letkovcs, I. and Pernis, B., eds.) at the Nco I restriction site (bases 120-151) and has the sequence:
SEQ ID No. 7:

This primer contains additional 7 bases at the 5' end to ensure a better binding of the Nco I restriction enzyme in the subsequent restriction enzyme cleavage. The second (forward) primer was complementary to bases 676-702 of the wild type gene and has the sequence:
SEQ ID No. 8:

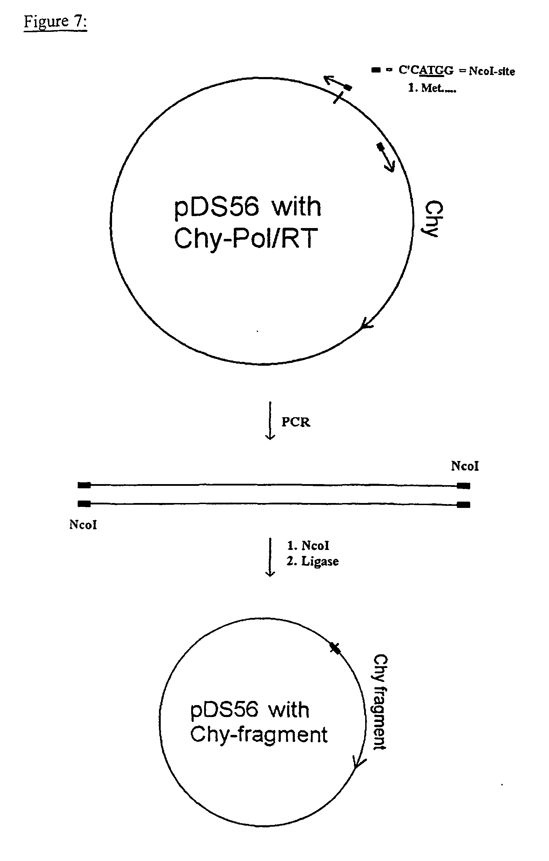

The forward primer also contained an additional Nco I restriction site and additional 7 bases at the 5'-end. Plasmid pDS56 DNA containing the polymerase-gene of *Carboxydothermus hydrogenoformans* at the Nco I/BamHI restriction sites was used as template for PCR. The PCR reaction was performed on the circular plasmid DNA pAR4. The fragment encoding the mutated *Carboxydothermus hydrogenoformans* DNA polymerase (Δ Chy) and the vector DNA were amplified as linear DNA by PCR using the Expand High Fidelity PCR System (Boehringer Mannheim) according to the supplier's specifications (Fig. 7). The length of the gene encoding Δ Chy is 1821 bp.
Amplification (Perkin Elmer GenAmp 9600 thermocycler) was carried out with the following conditions:
2 min 94 °C, (10 sec 94 °C; 30 sec 65 °C; 4 min 68 °C) x 10; (10 sec 94 °C; 30 sec 65 °C; 4 min 68 °C) + cycle elongation of 20 sec for each cycle) x 20; 7 min 72 °C;
After PCR the amplified DNA was purified using the High Pure PCR Product Purification Kit (Boehringer Mannheim) and digested with NcoI (3U / µg DNA) for 16 h (Boehringer Mannheim) according to the supplier's specifications.
For extraction with Phenol/Chloroform/Isoamylalcohol (24:24:1) the volume of the sample was raised to 100 µl with TE. After extraction the DNA was precipitated by adding 1/10 volumes of 3M Sodium Acetate, pH 5.2 and 2 volumes of EtOH. The DNA was circularized using the Rapid DNA Ligation Kit (Boehringer Mannheim) according to the supplier's specification. The ligated products were introduced into E. coli XL1-Blue by transformation according to the procedure of Chung, C. T. et al. (1989) *Proc. Natl. Acad. Sci.* USA **86**, 2172-2175. Transformants were plated on L-agar containing 100 µg/ml ampicillin to allow selection of recombinants. Colonies were picked and grown in L-broth containing 100 µg/ml ampicillin. Plasmid DNA was prepared with the High Pure Plasmid Isolation Kit (Boehringer Mannheim) according to the supplier's specification. The plasmids were screened for insertions by digestion with NcoI/BamHI. Strains containing the genes of interest were grown in L-broth supplemented with 100 µg/ml ampicillin and tested for the expression of DNA polymerase / reverse transcriptase activity by induction of exponentially growing culture with 1 mM IPTG and assaying the heat-treated extracts (72 °C) for DNA polymerase reverse transcriptase activity as described above (determination of DNA polymerase activity and determination of reverse transcriptase activity).

The present invention provides improved methods for efficiently transcribing RNA and amplifying RNA or DNA. These improvements are achieved by the discovery and application of previously unknown properties of thermoactive DNA polymerases with reverse transcriptase activity.

The enzyme of this invention may be used for any purpose in which such enzyme activity is necessary or desired. In a particularly preferred embodiment, the enzyme catalyzes reverse transcription of RNA which is amplified as DNA by a second DNA polymerase present in the amplification reaction known as RT-PCR (Powell et al. (1987) *Cell* **50**, 831-840). Any ribonucleic acid sequence, in purified or nonpurified form, can be utilized as the starting nucleic acid(s), provided it contains or is suspected to contain the specific nucleic acid sequence desired. The nucleic acid to be amplified can be obtained from any source, for example, from plasmids such as pBR322, from cloned RNA, from natural RNA from any source, including bacteria, yeast, viruses, organelles, and higher organisms such as plants and animals, or from preparations of nucleic acids made *in vitro.*

RNA may be extracted from blood, tissue material such as chorionic villi, or amniotic cells by a variety of techniques. See, e.g., Maniatis et al., (1982) Molecular Cloning: A Laboratory Manual (Cold Spring Harbor Laboratory, Cold Spring Harbor, New York) pp. 280-281. Thus the process may employ, for example, RNA, including messenger RNA, which RNA may be single-stranded or double-stranded. In addition, a DNA-RNA hybrid which contains one strand of each may be utilized.

The amplification of target sequences from RNA may be performed to proof the presence of a particular sequence in the sample of nucleic acid to be analyzed or to clone a specific gene. Δ Chy DNA polymerase is very useful for these processes. Due to its 3*'*-5*'* exonuclease activity it is able to synthesize products with higher accuracy as the reverse transcriptases of the state of the art.

Δ Chy DNA polymerase may also be used to simplify and improve methods for detection of RNA target molecules in a sample. In these methods Δ Chy DNA polymerase from *Carboxydothermus hydrogenoformans* may catalyze: (a) reverse transcription and (b) second strand cDNA synthesis. The use of DNA polymerase from *Carboxydothermus hydrogenoformans* may be used to perform RNA reverse transcription and amplification of the resulting complementary DNA with enhanced specificity and with fewer steps than previous RNA cloning and diagnostic methods.

Another aspect of the invention comprises a kit for performing RT-PCR comprising Δ Chy polymerase, reaction buffers, nucleotide mixtures, and optionally a thermostable DNA polymerase for detection and amplification of RNA either in a one step reaction or for reverse transcription of the template RNA and subsequent amplification of the cDNA product.

### Brief Description of the Drawings

Figure 1 shows the nucleic acid and amino acid sequence of the "Klenow fragment" of Chy polymerase designated Δ Chy.
Fig. 2 shows the reverse transcriptase activity of Δ Chy in dependence of magnesium and manganese salt.

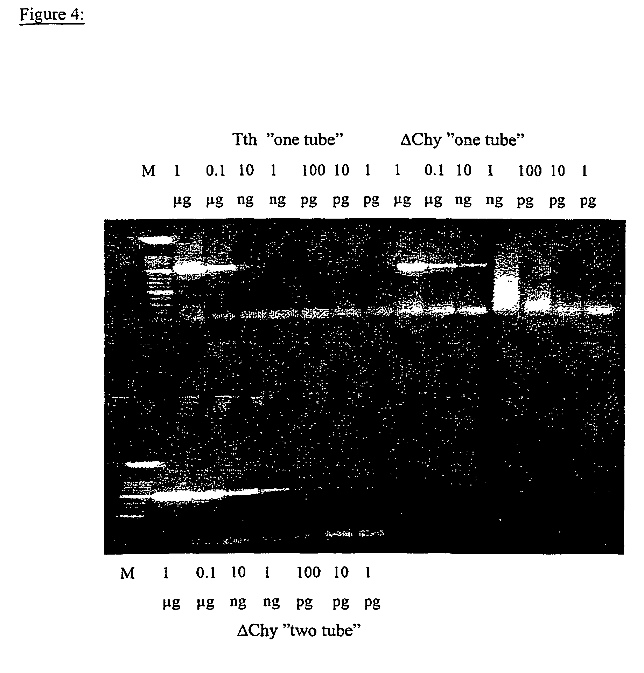

Figure 3 shows the reverse transcription and amplification of a 997 bp fragment of the β-Actin gene from total mouse liver RNA using Δ Chy and the Expand HiFi-System and decreasing amounts of RNA.
Figure 4 shows the reverse transcription and amplification of a 997 bp fragment of β-actin from total mouse liver RNA in comparison to Tth polymerase. Reverse transcription was either coupled with amplification ("one tube")
   using the Expand HiFi-System from Boehringer Mannheim, or after reverse transcription the Expand HiFi-System from Boehringer Mannheim was added to the reaction mixture for the subsequent amplification reaction ("two tube").

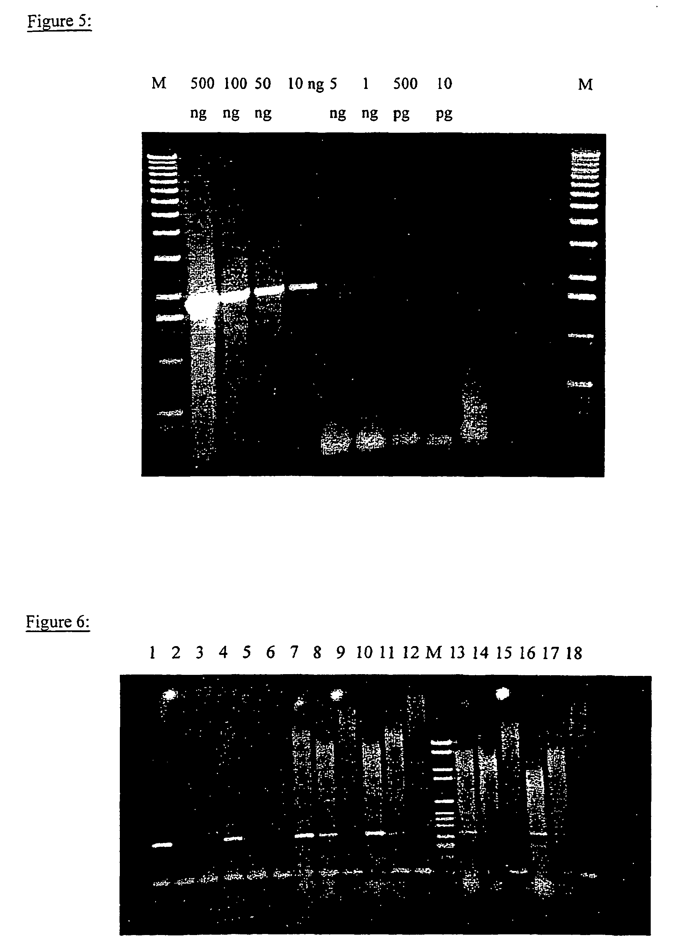

Figure 5 shows the reverse transcription and amplification of a 1,83 kb fragment of Dystrophin from total human muscle RNA.
Figure 6 shows the reverse transcription and amplification of a 324 bp fragment of β-actin from total mouse liver RNA with various amounts of Chy polymerase and various incubation times.
Figure 7 shows schematically the construction of the clone encoding Δ Chy from the clone encoding the wild type gene.

The following examples describe the invention in greater detail:

### Example 1

### Reverse transcription of a 324 bp β-actin fragment with Chy wild type DNA Polymerase used as Reverse Transcriptase followed by PCR with Taq-polymerase (Figure 6).

The reaction mixture (20 µl) contained 200 ng total mouse liver RNA, 200 µM dNTP, 10 mM Tris-HCl, pH 8.8, 5 mM DTT, 10 mM 2-mercaptoethanol, 15 mM KCl, 4.5 mM MgCl₂, 0.02 mg/ml BSA, 20 pmol of reverse primer (β-actin reverse: 5'-AAT TCG GAT GGC TAC GTA CAT GGC TG-3') and Chy-polymerase 33 units (lanes 1, 4, 7, 10, 13, 16), 13,2 units (lanes 2, 5, 8, 11, 14, 17) and 6,6 units (lanes 3, 6, 9, 12, 15, 18). Reactions were incubated for 5 min (lanes 1 to 6), 10 min (lanes 7 to 12) and 15 min (lanes 13 to 18) at 70 °C.
20 µl of the reverse transcription reaction was used as template for PCR (100 µl reaction volume) with Taq-polymerase (Boehringer Mannheim) according to the supplier's specification using 20 pmol of forward and reverse primer (Primer sequence "β-actin forward": 5'AGC TTG CTG TAT TCC CCT CCA TCG TG-3', primer sequence "β-actin reverse": 5'-AAT TCG GAT GGC TAC GTA CAT GGC TG-3') and 200 µM dNTP's.
Amplification was carried out using the following temperature profile:
2 min 94 °C; (10 sec 94 °C; 30 sec 60 °C; 30 sec 72 °C) x 30; 7 min 72 °C

### Example 2

### Construction of the vector expressing Δ Chy

The mutant was obtained by deletion of an N-terminal fragment of recombinant wild type *Carboxydothermus hydrogenoformans* DNA polymerase using inverse PCR (Innis et al., (1990) PCR Protocols; Academic Press, Inc., p 219-227). The reverse primer used is complementary to the cloning site of the expression vector pDS56 (Stüber, D., Matile, H. and Garotta, G. (1990) Immunological Methods, Letkovcs, I. and Pernis, B., eds.) at the Nco I restriction site (bases 120-151) and has the sequence: 5'-CGG TAA ACC CAT GGT TAA TTT CTC CTC TTT AAT GAA TTC-3'. This primer contains additional 7 bases at the 5' end to ensure a better binding of the Nco I restriction enzyme in the subsequent restriction enzyme cleavage. The second (forward) primer, was complementary to bases 676-702 of the wild type gene (sequence: 5'-CGG GAA TCC ATG GAA AAG CTT GCC GAA CAC GAA AAT TTA-3'). The forward primer also contained an additional Nco I restriction site and additional 7 bases at the 5'-end. Plasmid pDS56 DNA containing the polymerase-gene of *Carboxydothermus hydrogenoformans* at the Nco I/BamHI restriction sites was used as template for PCR. The PCR reaction was performed on circular plasmid DNA pAR4. The fragment of *Carboxydothermus hydrogenoformans* DNA polymerase (ΔChy) and the vector DNA were amplified as linear DNA by PCR using the Expand High Fidelity PCR System (Boehringer Mannheim) according to the supplier's specifications. The length of the gene encoding Δ Chy is 1821 bp.
Amplification (Perkin Elmer GeneAmp 9600 thermocycler) was carried out with the following conditions:
2 min 94 °C, (10 sec 94 °C; 30 sec 65 °C; 4 min 68 °C) x 10; (10 sec 94 °C; 30 sec 65 °C; 4 min 68 °C) + cycle elongation of 20 sec for each cycle) x 20; 7 min 72 °C;
After PCR the amplified DNA was purified using the High Pure PCR Product Purification Kit (Boehringer Mannheim) and digested with NcoI (3U / µg DNA) for 16 h (Boehringer Mannheim) according to the supplier's specifications.
For extraction with Phenol/Chloroform/Isoamylalcohol (24:24:1) the volume of the sample was raised to 100 µl with TE. After extraction the DNA was precipitated by adding 1/10 volumes of 3M Sodium Acetate, pH 5.2 and 2 volumes of EtOH. The DNA was circularized using the Rapid DNA Ligation Kit (Boehringer Mannheim) according to the supplier's specification. The ligated products were introduced into E. coli XL1-Blue by transformation according to the procedure of Chung, C. T. et al. (1989) *Proc. Natl. Acad. Sci.* USA **86**, 2172-2175. Transformants were plated on L-agar containing 100 µg/ml ampicillin to allow selection of recombinants. Colonies were picked and grown in L-broth containing 100 µg/ml ampicillin. Plasmid DNA was prepared with the High Pure Plasmid Isolation Kit (Boehringer Mannheim) according to the supplier's specification. The plasmids were screened for insertions by digestion with NcoI/BamHI. Strains containing the genes of interest were grown in L-broth supplemented with 100 µg/ml ampicillin and tested for the expression of DNA polymerase / reverse transcriptase activity by induction of exponentially growing culture with 1 mM IPTG and assaying the heat-treated extracts (72 °C) for DNA polymerase / reverse transcriptase activity as described above (determination of DNA polymerase activity and determination of Reverse Transcriptase activity). (Figure 7)

### Example 3

Reverse transcription and amplification of a 997 bp fragment of β-actin from total mouse liver RNA. Comparison of Δ Chy with Tth polymerase in the reverse transcription reaction (Figure 4) either in a coupled RT-PCR reaction ("one tube") or in consecutive steps, reverse transcription, addition of polymerase and amplification of the cDNA product of the first step.
"one tube" system:
   The reactions (50 µl) contained 10 mM Tris-HCl, pH 8.8 at 25 °C, 15 mM KCl, 2,5 mM MgCl₂, 400 µM of each dNTP, decreasing amounts of mouse total RNA (Clonetech) as indicated in the figure, 300 nM of each primer, 60 units of Δ Chy and 3,5 units of the Expand HiFi enzyme mix (Boehringer Mannheim GmbH). All reactions were incubated at 60 °C for 30 min (RT step). Amplification followed immediately with following cycle profile (Perkin Elmer GeneAmp 9600 thermocycler):
   30 sec. at 94 °C; (30 sec at 94 °C, 30 sec at 60 °C, 1 min. at 68 °C) x 10; (30 sec. at 94°C, 30 sec. at 60°C, 1 min. at 68°C + cyle elongation of 5 sec. for each cycle) x 20; 7 min at 68 °C;
"two tube" system:
   Reverse transcription is performed in 10 mM Tris-HCl, pH 8.8, 15 mM (NH₄)₂SO₄, 0.1 % Tween, 4,5 mM MgCl₂, 2 % DMSO, 800 µM dNTPs, 300 nmoles of each primer, 60 units of Δ Chy, various amounts of total mouse muscle RNA as indicated in the figure. The reaction was performed in a volume of 25 µl for 30 min at 60 °C.

5 µl of this reaction are used for the amplification with the Expand HiFi-system from Boehringer Mannheim. Amplification was performed with 2,6 units of polymerase mixture in a reaction volume of 25 µl. The following temperature cycling conditions were used: 30 sec. at 94°C, (30 sec. at 94°C, 30 sec at 60°C, 1 min at 68°C) x 10, (30 sec. at 94°C, 30 sec. at 60°C, 1 min at 68°C + cycle elongation for 5 sec for each cycle) X 20.

As a control reaction the same template-primer system was used for RT-PCR with Tth polymerase (Boehringer Mannheim). The reaction was set up according to the supplier's specifications for the "one step" variant.

### Literature cited:

Beckmann R. A. et al. (1985) *Biochemistry* **24**, 5810-5817
Berger et al., (1983) *Biochemistry* **22**, 2365-2372
Bessmann et al. (1957) *J. Biol. Chem.* **233**, 171-177
Braithwaite D. K. and Ito J. (1993) *Nucl. Acids Res.* **21**, 787 - 802
Brinkmann U. et al. (1989) *Gene* **85**, 109-114.
Brock et al. (1969) *J. Bacteriol.* **98**, 289-297
Buttin and Kornberg (1966) *J. Biol. Chem.* **241**, 5419-5427
Chung, C. T. et al. (1989) *Proc. Natl. Acad Sci.* USA **86**, 2172-2175
Engelke et al. (1990) *Anal. Biochem.* **191**, 396-400.
Gulati et al. (1974) *Proc. Nat. Acad Sci.* USA **71**, 1035-1039
Höltke, H.-J.; Sagner, G; Kessler, C. and Schmitz, G. (1992) *Biotechniques* **12**, 104 -113.
Innis et al., (1990) PCR Protocols; Academic Press, Inc., 219-227
Kaledin et al. (1980), *Biokhimiya* **45**, 644-651.
Kaledin et al. (1981) *Biokhimiya* **46**, 1576-1584.
Kaledin et al. (1982) *Biohkimiya* **47**, 1785-1791.
Karkas (1973) *Proc. Nat. Acad. Sci.* USA **70**, 3834-3838
Kornberg A. and Baker T.A.(1992) *DNA Replication* W. H. Freemann & Company, New York.
Lawyer et al. (1989) *J. Biol. Chem.* **264**, 6427-6437.
Lundberg et al. (1991) *Gene* **108**, 1-6.
Maniatis et al. (1982) *Molecular Cloning: A Laboratory Manual*, Cold Spring Harbor, New York.
Neuner et al. (1990) *Arch. Microbiol.* **153**, 205-207.
Powell et al. (1987) *Cell* **50**, 831-840
Perbal (1984), A Practical Guide to Molecular Cloning, Wiley & Sons New York
Perler et al. (1992) *Proc. Natl. Acad. Sci.* USA **89**, 5577-5581.
Powell et al. (1987) *Cell* **50**, 831-840
Ricchetti M. and Buc H. (1993) *EMBO J.* **12**, 387-396.
Ruttimann et al. (1985) *Eur. J. Biochem.* **149**, 41-46.
Saunders and Saunders (1987) Microbial Genetics Applied to Biotechnology, Croom Helm, London
Spanos A. and Hübscher U. (1983) *Methods in Enzymology* **91**, 263-277.
Stüber D., Matile H. and Garotta G. (1990) *Immunological Methods*, Letkovcs, I and Pernis, B., eds.
Svetlichny et al. (1991) *System. Appl. Microbiol.*, **14**, 205-208.
Triglia T. et al. (1988) *Nucleic Acids Res.* **16**, 8186.
Verma (1977) *Biochem. Biophys. Acta* **473**, 1
Wittig and Wittig, (1978) *Nuc. Acids Res.* **5**, 1165-1178

## Claims

1. A purified DNA polymerase obtainable from *Carboxydothermus hydrogenoformans* exhibiting reverse transcriptase activity in the presence of magnesium ions and/or manganese ions, having reduced or no 5'-3'-exonuclease activity, having substantially no RNaseH activity, and having amino acid sequence SEQ ID NO.: 11.

2. A DNA polymerase according to claim 1, wherein the reverse transcriptase activity in the presence of magnesium ions is higher than the reverse transcriptase activity in the presence of manganese ions of said polymerase.

3. A DNA polymerase as claimed in any of claims 1-2, wherein said polymerase is a mutant with reduced or no 5*'*-3*'*exonuclease activity derived from a naturally occurring polymerase possessing 5*'*-3*'*exonuclease activity.

4. A DNA polymerase as claimed in any of claims 1-3, wherein said polymerase has an apparent molecular weight between about 64 to 71 kDa as determined by SDS polyacrylamide electrophoresis.

5. A recombinant DNA polymerase as claimed in any of claims 1-4, wherein said polymerase is obtainable from E. coli, the strain being designated E.coli GA1 (DSM No. 11854).

6. An isolated DNA sequence coding for the polymerase as claimed in any one of claims 1-5.

7. A recombinant DNA sequence capable of encoding a DNA polymerase as claimed in any one of claims 1-5.

8. An isolated DNA sequence represented by the formula shown in SEQ ID No. 10.

9. A vector containing the isolated DNA sequence as claimed in any of claims 6-8.

10. A vector according to claim 9, wherein such vector is plasmid pDS56 carrying a deletion mutant of the *Carboxydothermus hydrogenoformans* DNA polymerase gene.

11. The vector according to claim 9 providing some or all of the following features:
(1) promotors or sites of initiation of transcription
(2) operators which could be used to turn gene expression on or off
(3) ribosome binding sites for improved translation
(4) transcription or translation termination sites

12. A microbial host transformed with the vector of claims 9-11.

13. A microbial host according to claim 12, wherein said transformant is E. coli, the strain being designated E.coli GA1 (DSM No. 11854).

14. A process for the preparation of DNA polymerase according to any of the claims 1-5 comprising the steps:
(a) culturing the natural strain *Carboxydothermus hydrogenoformans*
(b) suspending the cells of the natural strain in buffer
(c) disrupting the cells
(d) purifying the DNA polymerase by chromatographic steps including the use of one or more Sepharose-columns.

15. A process for the preparation of DNA polymerase according to any one of claims 1-5 comprising growing a recombinant E. coli strain transformed with a vector according to claims 9-11 and purifying and isolating the DNA polymerase.

16. A process of amplifying RNA, **characterized in that** a thermophilic DNA polymerase as claimed in any one of claims 1-5 is used in combination with a thermostable DNA polymerase.

17. A process for cDNA cloning and DNA sequencing, **characterized in that** a thermophilic DNA polymerase as claimed in any one of claims 1-5 is used.

18. A process for DNA labeling, **characterized in that** a thermophilic DNA polymerase as claimed in any one of claims 1-5 is used.

19. A process for reverse transcription of RNA to cDNA **characterized in that** a thermophilic DNA polymerase as claimed in any one of claims 1-5 is used.

20. A kit useful for RT-PCR comprising reverse transcription of RNA using a thermophilic DNA polymerase as claimed in any one of claims 1-5 and amplification of the cDNA product by a thermostable DNA polymerase either in a combined reaction (RT and PCR) or for consecutive reactions (RT and subsequently PCR) wherein said kit comprises a DNA polymerase as claimed in anyone of claims 1-5.

## Patentansprüche

1. Eine gereinigte DNA-Polymerase erhältlich von *Carboxydothermus hydrogenoformans*, welche in der Gegenwart von Magnesium-Ionen und/oder Mangan-Ionen eine reverse Transkriptase-Aktivität aufweist, mit reduzierter oder keiner 5' -3' -Exonuklease-Aktivität, mit praktisch keiner RNaseH-Aktivität und mit Aminosäure-Sequenz SEQ ID NO.: 11.

2. DNA-Polymerase gemäß Anspruch 1, bei der die reverse Transkriptase-Aktivität der genannten Polymerase in der Gegenwart von Magnesium-Ionen höher ist als die reverse Transkriptase-Aktivität in der Gegenwart von Mangan-Ionen.

3. DNA-Polymerase gemäß einem der Ansprüche 1 - 2, in der die genannte Polymerase eine Mutante mit reduzierter oder keiner 5'-3'-Exonuklease-Aktivität ist, die von einer natürlich vorkommenden Polymerase, die 5' -3' -Exonuklease-Aktivität aufweist abgeleitet ist.

4. DNA-Polymerase gemäß einem der Ansprüche 1 - 3, in der die genannte Polymerase ein scheinbares Molekulargewicht zwischen ungefähr 64 bis 71 kDa, wie mittels SDS-Polyacrylamid-Elektrophorese bestimmt, aufweist.

5. Eine rekombinante DNA-Polymerase gemäß einem der Ansprüche 1 - 4, in der die genannte Polymerase erhältlich ist von E.coli und der Stamm mit E.coli GA1 (DSM No. 11854) **gekennzeichnet** ist.

6. Eine isolierte DNA-Sequenz, welche für die Polymerase gemäß einem der Ansprüche 1 - 5 kodiert.

7. Eine rekombinante DNA-Sequenz, welche in der Lage ist, eine Polymerase gemäß einem der Ansprüche 1 - 5 zu kodieren.

8. Eine isolierte DNA-Sequenz, welche durch die in SEQ ID No. 10 gezeigte Formel dargestellt ist.

9. Ein Vektor, der die isolierte DNA-Sequenz gemäß einem der Ansprüche 6 - 8 enthält.

10. Vektor gemäß Anspruch 9, wobei dieser Vektor Plasmid pDS56 ist, das eine Deletionsmutante der *Carboxydothermus hydrogenoformans* DNA-Polymerase Gene trägt.

11. Vektor gemäß Anspruch 9, welcher einige oder alle der folgenden Merkmale bereitstellt:
(1) Promotoren oder Stellen der Initiierung der Transkription
(2) Operatoren, welche verwendet werden können, um die Genexpression ein- oder abzuschalten
(3) Ribosom-bindende Stellen zur verbesserten Translation
(4) Transkriptions- oder Translations-Terminationsstellen.

12. Ein mikrobieller Wirt, der mit dem Vektor gemäß der Ansprüche 9 - 11 transformiert ist.

13. Mikrobieller Wirt gemäß Anspruch 12, wobei die genannte Transformante E.coli ist und der Stamm mit E.coli GA1 (DSM No. 11854) **gekennzeichnet** ist.

14. Ein Verfahren zur Herstellung von DNA-Polymerase gemäß einem der Ansprüche 1 - 5, umfassend die Schritte:
(a) Kultivieren des natürlichen Stamms *Carboxydothermus hydrogenoformans*
(b) Suspendieren der Zellen des natürlichen Stamms in einem Puffer
(c) Zerstören der Zellen
(d) Reinigen der DNA-Polymerase mittels Chromatographieschritten, die die Verwendung von einer oder mehreren Sepharose-Säulen beinhaltet.

15. Verfahren zur Herstellung von DNA-Polymerase gemäß einem der Ansprü che 1 - 5, umfassend das Wachsen eines rekombinanten E.coli Stamms, der mit einem Vektor gemäß der Ansprüche 9 - 11 transformiert ist und Reinigung und Isolierung der DNA-Polymerase.

16. Ein Verfahren zur Ampflifikation von RNA, **dadurch gekennzeichnet, dass** eine thermophile DNA-Polymerase gemäß einem der Ansprüche 1 - 5 in Kombination mit einer thermostabilen DNA-Polymerase verwendet wird.

17. Ein Verfahren zur Klonierung von cDNA und zur Sequenzierung von DNA, **dadurch gekennzeichnet, dass** eine thermophile DNA-Polymerase gemäß einem der Ansprüche 1 - 5 verwendet wird.

18. Ein Verfahren zur Markierung von DNA, **dadurch gekennzeichnet, dass** eine thermophile DNA-Polymerase gemäß einem der Ansprüche 1 - 5 verwendet wird.

19. Ein Verfahren zur reversen Transkription von RNA zu cDNA, **dadurch gekennzeichnet, dass** eine thermophile DNA-Polymerase gemäß einem der Ansprüche 1 - 5 verwendet wird.

20. Ein Kit geeignet für RT-PCR, umfassend eine reverse Transkription von RNA unter Verwendung einer thermophilen DNA-Polymerase gemäß einem der Ansprüche 1 - 5 und Ampflifikation des cDNA-Produkts durch eine thermostabile DNA-Polymerase entweder in einer kombinierten Reaktion (RT und PCR) oder in nachgeschalteten Reaktionen (RT und anschließ end PCR), wobei das genannte Kit eine DNA-Polymerase gemäß einem der Ansprüche 1 - 5 umfasst.

## Revendications

1. ADN polymérase purifiée, pouvant être obtenue à partir de *Carboxydothermus hydrogenoformans*, manifestant une activité transcriptase inverse en présence d'ions magnésium et/ou d'ions manganèse, ayant une activité 5'-3' exonucléase réduite ou nulle, n'ayant pratiquement aucune activité RNaseH, et possédant la séquence d'acides aminés SEQ ID NO. : 11.

2. ADN polymérase selon la revendication 1, dans laquelle l'activité transcriptase inverse en présence d'ions magnésium est supérieure à l'activité transcriptase inverse en présence d'ions manganèse de ladite polymérase.

3. ADN polymérase selon l'une quelconque des revendications 1 à 2, ladite polymérase étant un mutant ayant une activité 5'-3' exonucléase réduite ou nulle, dérivée d'une polymérase naturellement présente et possédant une activité 5'-3' exonucléase.

4. ADN polymérase selon l'une quelconque des revendications 1 à 3, ladite polymérase ayant une masse moléculaire apparente comprise entre environ 64 et 71 kDa telle que déterminée par électrophorèse sur gel de SDS polyacrylamide.

5. ADN polymérase recombinante selon l'une quelconque des revendications 1 à 4, ladite polymérase pouvant être obtenue à partir de E. coli, la souche étant intitulée E. coli GA1 (DSM No. 11854).

6. Séquence d'ADN isolée codant pour la polymérase selon l'une quelconque des revendications 1 à 5.

7. Séquence d'ADN recombinée capable de coder pour une ADN polymérase selon l'une quelconque des revendications 1 à 5.

8. Séquence d'ADN isolée représentée par la formule montrée dans SEQ ID No. 10.

9. Vecteur contenant la séquence d'ADN isolée selon l'une quelconque des revendications 6 à 8.

10. Vecteur selon la revendication 9, ledit vecteur étant un plasmide pDS56 portant un mutant par délétion du gène de l'ADN polymérase de *Carboxydothermus hydrogenoformans*.

11. Vecteur selon la revendication 9, fournissant certaines ou toutes les caractéristiques suivantes :
(1) des promoteurs ou des sites d'initiation de la transcription
(2) des opérateurs qui pourraient être utilisés pour activer ou éteindre l'expression du gène
(3) des sites de liaison sur les ribosomes permettant une amélioration de la traduction
(4) des sites de terminaison de la transcription ou de la traduction.

12. Hôte microbien transformé avec le vecteur selon les revendications 9 à 11.

13. Hôte microbien selon la revendication 12, chez lequel ledit transformant est E. coli, la souche étant intitulée E. coli GA1 (DSM No. 11854).

14. Procédé de préparation d'une ADN polymérase selon l'une quelconque des revendications 1 à 5, comprenant les étapes de :
(a) culture de la souche naturelle *Carboxydothermus hydrogenoformans*
(b) mise en suspension des cellules de la souche naturelle dans un tampon
(c) lyse des cellules
(d) purification de l'ADN polymérase par l'intermédiaire d'étapes de chromatographie comprenant l'utilisation d'une ou plusieurs colonnes de Sepharose.

15. Procédé de préparation d'une ADN polymérase selon l'une quelconque des revendications 1 à 5, comprenant la croissance d'une souche de E. coli recombinante, transformée avec un vecteur selon les revendications 9 à 11, et la purification et l'isolement de l'ADN polymérase.

16. Procédé d'amplification d'un ARN, **caractérisé en ce qu'**une ADN polymérase thermophile selon l'une quelconque des revendications 1 à 5 est utilisée en combinaison avec une ADN polymérase thermostable.

17. Procédé de clonage d'ADNc et de séquençage d'ADN, **caractérisé en ce qu'**une ADN polymérase thermophile selon l'une quelconque des revendications 1 à 5 est utilisée.

18. Procédé de marquage d'un ADN, **caractérisé en ce qu'**une ADN polymérase thermophile selon l'une quelconque des revendications 1 à 5 est utilisée.

19. Procédé de transcription inverse d'un ARN en ADNc, **caractérisé en ce qu'**une ADN polymérase thermophile selon l'une quelconque des revendications 1 à 5 est utilisée.

20. Trousse utile pour une RT-PCR comprenant la transcription inverse d'un ARN en utilisant une ADN polymérase thermophile selon l'une quelconque des revendications 1 à 5 et l'amplification du produit ADNc par une ADN polymérase thermostable soit dans une réaction combinée (RT et PCR) soit pour des réactions successives (RT et PCR ultérieure), ladite trousse comprenant une ADN polymérase selon l'une quelconque des revendications 1 à 5.
